# EUROPÄISCHE PATENTANMELDUNG

(11) **EP 0 949 503 A1**
(43) Veröffentlichungstag der Anmeldung: **13.10.1999**
(21) Anmeldenummer: 99104897.6
(22) Anmeldetag: 11.03.1999
(51) Int. Cl.: G01N 21/66, G01N 21/76

(54) **Verfahren zum Nachweis eines Analyten in einer Probe durch Elektrochemilumineszenzmessung**

(30) Priorität: 17.03.1998 DE 19811582
(71) Anmelder: Roche Diagnostics GmbH, 68298 Mannheim (DE)
(72) Erfinder: Punzmann, Gabriele, 81547 München (DE); Josel, Hans-Peter, 82362 Weilheim (DE); Egger, Martin, Dr., 82347 Bernried (DE)
(74) Vertreter: Weiss, Wolfgang, Dipl.-Chem. Dr.

(57) **Zusammenfassung**

Die Erfindung betrifft die Stabilisierung und Verstärkung von Elektrochemilumineszenzsignalen in einem Verfahren zum Nachweis eines Analyten in einer Probe durch Elektrochemilumineszenzmessung, umfassend die Schritte:
(a) Bereitstellen einer Elektrochemilumineszenzvorrichtung umfassend eine Meßelektrode,
(b) Inkontaktbringen einer Konditionierungsflüssigkeit, die ein Elektronchemilumineszenz-Cosubstrat enthält, mit der Elektrode,
(c) Einstellen von Bedingungen an der Elektrode, welche zur Ausbildung einer aktivierte Moleküle des Elektrochemilunineszenz-Cosubstrats enthaltenden Schicht auf oder/und im Grenzschichtbereich der Elektrode führen,
(d) Inkontaktbringen der Probe, die einen Metallkomplex, der mindestens einen Ladungsträger oder/und mindestens eine hydrophile Gruppe enthält, als Elektrochemilumineszenz-Markierungsgruppe sowie ein Elektrochemilumineszenz-Cosubstrat enthält, mit der Elektrode,
(e) Anlegen eines Potentials an die Elektrode, das den Ablauf einer Elektrochemilumineszenzreaktion ermöglicht, und Messen der Elektrochemilumineszenz und
(f) Korrelieren der gemessenen Lumineszenz mit dem Vorhandensein bzw. der Menge des Analyten in der Probe.

## Beschreibung

Die Erfindung betrifft die Stabilisierung und Verstärkung von Elektrochemilumineszenzsignalen in Nachweisverfahren.

Lumineszierende Metallkomplexe sind aus dem Stand der Technik bekannt. EP-A-0 178 450 offenbart Rutheniumkomplexe, die an ein immunologisch aktives Material gekoppelt sind, wobei die Rutheniumkomplexe drei gleiche oder verschiedene bi- oder polycyclische Liganden mit mindestens zwei stickstoffhaltigen Heterocyclen enthalten, wobei mindestens einer dieser Liganden mit mindestens einer wasserlöslich-machenden Gruppe wie -SO₃H oder -COOH substituiert ist und wobei mindestens einer dieser Liganden mit mindestens einer reaktiven Gruppe wie -COOH direkt oder über eine Spacergruppe substituiert ist und wobei die Liganden über Stickstoffatome an das Ruthenium gebunden sind.

Weiterhin ist die Verwendung von Metallkomplexen als Markierungsreagenzien für ein Elektrochemilumineszenz-Nachweisverfahren bekannt (vgl. z.B. EP-A-0 580 979, WO 87/06706, US 5,238,108 oder US 5,310,687). Ein solches Elektrochemilumineszenz-Nachweisverfahren beruht darauf, daß in einer geeigneten Meßvorrichtung das Zentralatom des Metallkomplexes, z.B. Ruthenium, durch Elektronentransfer in den angeregten MLCT-Triplettzustand überführt wird. Von diesem angeregten Zustand kann es unter Emission eines Photons durch einen verbotenen Triplett-Singulett-Übergang in den Grundzustand relaxieren (vgl. z.B. WO/90 05296, Leland und Powell, J. Electrochem. Soc. 137 (1990), 3127-3131; Blackburn et al., Clin. Chem. 37 (1991), 1534-1539).

Ein Nachteil dieses Verfahrens besteht darin, daß die maximal erhältliche Höhe des Meßsignals aufgrund eines ausgeprägten Abklingens der Signalintensität während der Meßphase sehr begrenzt ist. Bei der bisher üblichen Verfahrensführung und den bisher kommerziell verwendeten Metallkomplexen, insbesondere Ruthenium-(Bipyridyl)₃-Komplexen, tritt dieses Abklingen des Signals bereits nach einer Meßdauer von 100 ms auf und nimmt mit der Signalstärke zu. Dieses Verhalten ist anhand bisheriger Publikationen zum Reaktionsmechanismus nicht verständlich. Aufgrund dieses Signalabfalls wird die Dauer des Meß- und Auswerteintervalls ganz erheblich, d.h. in der Praxis auf maximal 400 ms begrenzt. In allen bisherigen Fällen führt dies zu einer signifikant verringerten Lichtausbeute, die wiederum eine Verringerung der Testsensitivtät und -dynamik zur Folge hat. In der Praxis können deshalb nur Meß- und Auswerteintervalle von maximal 400 ms genutzt werden, ohne Probleme mit unspezifischen Signalen zu erhalten. Darüber hinaus treten im abklingenden Teil des Signalverlaufs häufig Impräzisionen und Signalinstabilitäten auf, die zu weiteren Ungenauigkeiten führen.

In WO 96/03409 und WO 96/03410 werden neue Metallkomplexe mit hydrophilen Substituenten oder/und Ladungsträgern am Linker beschrieben. Durch Verwendung dieser Komplexe wird eine Verringerung einer unerwünschten Adsorption erreicht, wobei die Stabilität und Wiederfindung im Nachweisverfahren verbessert wird. Weiterhin wird eine erhöhte Quantenausbeute beschrieben. Es finden sich jedoch keine Angaben über eine mögliche Verlängerung des maximalen Meßintervalls bei Elektrochemilumineszenz-Messungen.

Überraschenderweise wurde festgestellt, daß bei Verwendung von hydrophilen oder/und geladenen Metallkomplexen, beispielsweise gemäß EP-A-0 178 450, WO 96/03409 oder WO 96/03410 wesentliche Verbesserungen für den Elektrochemilumineszenznachweis erzielt werden können, da der bei nichthydrophilen Ruthenium-(Bipyridin)₃-Komplexen bekannte Signalabfall nicht auftritt. Das Signal behält überraschenderweise seinen Maximalwert im wesentlichen über die Gesamtdauer des Meßintervalls bei. Dies führt zu einer Signalverstärkung oder/und zu einer Erhöhung der Dauer des maximal möglichen Meßintervalls. Diese Verbesserung wird vorzugsweise unter Verfahrensbedingungen erreicht, bei denen vor der Messung an die Meßelektrode ein negatives Potential in Gegenwart des Elektrochemilumineszenz-Cosubstrats angelegt wird.

Ein Gegenstand der Erfindung ist somit ein Verfahren zum Nachweis eines Analyten in einer Probe durch Elektrochemilumineszenzmessung, umfassend die Schritte:
(a) Bereitstellen einer Elektrochemilumineszenzvorrichtung umfassend eine Meßelektrode,
(b) Inkontaktbringen einer Konditionierungsflüssigkeit, die ein Elektrochemilumineszenz-Cosubstrat enthält, mit der Elektrode,
(c) Einstellen von Bedingungen an der Elektrode, welche zur Ausbildung einer aktiverte Moleküle des Elektrochemilumineszenz-Cosubstrats enthaltenden Schicht auf oder/und im Grenzschichtbereich der Elektrode führen, z.B. durch Anlegen eines negativen Potentials an die Elektrode,
(d) Inkontaktbringen der Probe, die einen Metallkomplex, der mindestens einen Ladungsträger oder/und mindestens eine hydrophile Gruppe enthält, als Elektrochemilumineszenz-Markierungsgruppe sowie ein Elekrochemilumineszenz-Cosubstrat enthält, mit der Elektrode,
(e) Anlegen eines Potentials an die Elektrode, welches den Ablauf einer Elektrochemilumineszenzreaktion ermöglicht, und Messen der Elektrochemilumineszenz und
(d) Korrelieren der gemessenen Lumineszenz mit dem Vorhandensein bzw. der Menge des Analyten in der Probe.

Durch das erfindungsgemäße Verfahren wird aufgrund des fehlenden Signalabfalls während des Meßintervalls ein um mindestens den Faktor 2 bis 5 höheres Meßsignal als bei einer üblichen nichthydrophilen Ruthenium-(Bipyridyl)₃-Markierungsgruppe erhalten. Diese höhere Signalstärke ermöglicht den Einsatz preiswerterer Halbleiter-Detektoren statt der bisher verwendeten Photomultiplyer-Röhren. Weiterhin kann unter geeigneten Meßbedingungen, d.h. bei Aufrechterhalten eines für die Lumineszenzreaktion ausreichenden positiven Potentials und gleichzeitigem ausreichenden Angebot an Elektrochemilumineszenz-Cosubstrat, über ein beliebig langes Zeitintervall eine pro Zeiteinheit konstant bleibende Lichtmenge erzeugt werden. Auf diese Weise kann eine viel größere Lichtmenge gesammelt und höhere Testsensitivitäten erzielt werden.

Weitere Vorteile der erfindungsgemäß verwendeten Metallkomplexe bestehen darin, daß ein geringeres Quenching durch Sauerstoff auftritt und daß geringere Teststörungen, z.B. verursacht durch unspezifische Adsorption an Testkomponenten oder/und die Elektrode, erzielt werden.

Die im erfindungsgemäßen Verfahren gemäß Schritt (a) bereitgestellte Elektrochemilumineszenz-Meßvorrichtung kann eine bekannte Vorrichtung des Standes der Technik sein (vgl. beispielsweise N.R. Hoyle: "The Application of Electrochemilumenscence to Immunoassay-based Analyte Measurement", in: "Bioluminescence and Chemiluminescence"; Proceedings of the 8th International Symposium on Bioluminescence and Chemiluminescence, Cambridge, September 1994, A. K. Campbell et al. (Hrsg.), John Wiley & Sous; WO 89/10551; WO 90/11511). Die Vorrichtung umfasst vorzugsweise eine Meßkammer zur Aufnahme der Meßelektrode, Mittel zur Zufuhr und zur Ableitung von Flüssigkeiten aus der Meßkammer und Mittel zum Nachweis der in der Meßkammer erzeugten Elektrochemilumineszenz. Weiterhin enthält die Vorrichtung vorzugsweise magnetische Mittel zur Immobilisierung magnetischer Partikel in der Probeflüssigkeit an der Meßelektrode.

Schrift (b) des Verfahrens umfasst das Inkontaktbringen der Elektrode mit einer Konditionierungsflüssigkeit, die ein als Oxidations- oder Reduktionsmittel für den Metallkomplex wirksames Elektrochemilumineszenz-Cosubstrat, z.B. ein Amin oder ein Persulfat enthält. Vorzugsweise werden tertiäre Amine z.B. Trialkylamine verwendet, wobei die Alkylreste jeweils unabhängig jeweils 1-4 C-Atome enthalten. Besonders bevorzugt ist Tripropylamin. Die Konzentration des Cosubstrats in der Konditiontierungsflüssigkeit kann über weite Bereiche variiert werden, sie beträgt vorzugsweise mindestens 1 mM, besonders bevorzugt 10 bis 500 mM und am meisten bevorzugt 100 bis 300 mM. Weiterhin kann die Konditionierungsflüssigkeit einen geeigneten elektrochemisch inerten Puffer, z.B. einen Phosphatpuffer etc, und ein Detergens, z.B. Thesit, enthalten.

Gemäß Schrift (c) werden an der Elektrode Bedingungen eingestellt, bei denen eine Anlagerung von aktivierten, insbesondere reduzierten Molekülen des Cosubstrats stattfindet. Die Anlagerung kann als Adsorption, aber auch durch Ausbildung einer die Cosubstratmoleküle enthaltenden Grenzschicht in unmittelbarer Nähe der Elektrodenoberfläche erfolgen. Vorzugsweise wird hierzu ein negatives Potential an die Elektrode angelegt, vorzugsweise in Anwesenheit der Konditionierungsflüssigkeit. Die Höhe des negativen Potentials ist vorzugsweise mindestens -0,3 V, bespielsweise -1,2 bis -1,0 V. Die Dauer des Anlegens des negativen Potentials ist vorzugsweise 0,2 bis 20 s, besonders bevorzugt ca. 0,5 s.

Schritt (d) des erfindungsgemäßen Verfahrens umfaßt das Inkontaktbringen der Probe mit der Meßelektrode. Die Probe ist vorzugsweise eine biologische Probe und liegt in flüssiger Form vor. Sie kann aus menschlichen, tierischen oder pflanzlichen Geweben, Körperflüssigkeiten, prokaryontischen oder eukaryontischen Zellkulturen etc. stammen. Dieser Probe werden die zur Bestimmung des jeweiligen Analyten benötigten Nachweisreagenzien zugesetzt. Diese Nachweisreagenzien umfassen einen elektrochemilumineszierenden Metallkomplex wie im folgenden definiert als Markierungsgruppe, der vorzugsweise an eine biologische Substanz, z.B. Biotin, Nukleinsäuren, z. B. Oligonukleotide, DNA oder RNA, Nukleinsäureanaloga wie etwa peptidische Nukleinsäuren, Antikörper oder Antikörperfragmente, Polypeptidantigene, d.h. immunologisch reaktive Polypeptide oder Haptene, d.h. organische Moleküle mit einem Molekulargewicht von 150 bis 2000 gekoppelt ist, sowie gegebenenfalls weitere Nachweisreagenzien, wie sie dem Fachmann bekannt sind. Weiterhin enthält die Probe ein Elektrochemilumineszenz-Cosubstrat wie zuvor definiert.

Das erfindungsgemäße Verfahren kann als homogener Assay, d.h. Messung der Elektrochemilumineszenz in der Flüssigphase, durchgeführt werden. Vorzugsweise wird jedoch ein heterogener Test durchgeführt, bei dem die Elektrochemilumineszenz-Markierung an einer Festphase, z.B. einer partikulären Festphase wie etwa magnetischen Mikrobeads, z.B. Streptavidin-beschichteten Mikrobeads, oder kolloidalen Partikeln immobilisiert wird. Bei Durchführung eines heterogenen Tests umfaßt das erfindungsgemäße Verfahren sog. Einfang- und Waschschritte, bei denen die Festphase an der Elektrode immobilisiert wird und eine Abtrennung der übrigen Probenbestandteile erfolgt.

Zur Messung der Elektrochemilumineszenz wird gemäß Schritt (e) ein Potential an die Elektrode angelegt, welches das Auftreten einer Elektrochemilumineszenzreaktion ermöglicht, d.h. ein für die elektrochemilumineszenten Metallkomplexe und die Cosubstrate oxidatives Potential, und die Elektrochemilumineszenz bestimmt. Das oxidative Elektrodenpotential beträgt vorzugsweise mindestens + 1,2 V (bezogen auf eine Ag/AgCl-Referenzelektrode). Insbesondere im Fall von hydrophilen oder/und geladenen Ruthenium-bathophenanthrolin-Komplexen werden Meßspannungen von mindestens + 1,4 V, z.B. zwischen + 1,4 V und + 2,4 V, besonders bevorzugt. Die Dauer des Meßintervalls kann aufgrund des nichtauftretenden Signalabfalls gegenüber der bisher üblichen Meßdauer deutlich verlängert werden. Vorzugsweise beträgt das Meßintervall mindestens 0,5 s, besonders bevorzugt mindestens 1 s und am meisten bevorzugt mindestens 2 s. Dabei wird vorzugsweise während der gesamten Dauer des Meßintervalls ein oxidatives Potential angelegt, welches ausreicht, die Lumineszenzreaktion aufrechtzuerhalten. Besonders bevorzugt wird während der gesamten Meßdauer ein im wesentlichen konstantes oxidatives Potential an die Elektrode angelegt.

Schritt (f) des erfindungsgemäßen Verfahrens umfaßt das Korrelieren der gemessenen Elektrochemilumineszenz mit dem Vorhandensein bzw. der Menge des zu bestimmenden Analyten in der Probe. Auf diese Weise kann eine qualitative oder/und quantitative Bestimmung von Analyten durch übliche dem Fachmann bekannte Methoden erfolgen.

Ein wesentliches Merkmal des erfindungsgemäßen Verfahrens ist die Verwendung von elektrochemilumineszenten Metallkomplexen, die einen Liganden mit einem Ladungsträger oder/und mindestens einer hydrophilen Gruppe enthalten. Bevorzugt wird ein Metallkomplex als Markierungsgruppe verwendet, der eine Struktur der allgemeinen Formel (I) enthält:

[M(L₁L₂L₃)]ₙ-Yₘ- (I)

worin
- M ein zwei- oder dreiwertiges Metallkation ausgewählt aus Seltenerde- oder Übergangsmetallkationen ist,
- L₁, L₂ und L₃ gleich oder verschieden sind und Liganden mit mindestens zwei stickstoffhaltigen Heterocyclen bedeuten, wobei L₁, L₂ und L₃ über Stickstoffatome an das Metallkation gebunden sind,
- Y einen an einen der Liganden gebundenen Linker bedeutet, über den der Komplex, z.B. (a) an eine biologische Substanz gekoppelt ist, oder (b) an eine biologische Substanz gekoppelt werden kann,
- m eine ganze Zahl von 1 bis 10, vorzugsweise von 1 bis 4 und besonders bevorzugt von 1 ist,
- n eine ganze Zahl von 1 bis 6 vorzugsweise 1 bis 3 und besonders bevorzugt von 1 ist, und
- mindestens eine hydrophile Gruppe oder/und ein Ladungsträger im Komplex vorhanden ist.

Das Metallkation in diesem Komplex ist vorzugsweise Ruthenium, Osmium, Rhenium, Iridium, Rhodium, Platin, Indium, Palladium, Molybdän, Techneticum, Kupfer, Chrom, Wolfram, Yttrium oder Lutetium. Besonders bevorzugt sind Ruthenium, Iridium, Rhenium, Chrom und Osmium. Am meisten bevorzugt ist Ruthenium. Zum Ladungsausgleich kann der Komplex gegebenenfalls noch Gegenionen, z.B. Anionen, enthalten.

Die Liganden L₁, L₂ und L₃ sind vorzugsweise Liganden mit mindestens 2 stickstoffhaltigen Heterocyclen. Bevorzugt sind aromatische Heterocyclen wie z.B. Bipyridyl, Bipyrazyl, Terpyridyl und Phenanthronyl. Besonders bevorzugt werden die Liganden aus Bipyridin- und Phenanthrolin-Ringsystemen ausgewählt. Am meisten bevorzugt enthalten die Liganden Bathophenanthrolin-Ringsysteme.

In den erfindungsgemäßen Metallkomplexen sind die hydrophilen Gruppen oder/und die Ladungsträger vorzugsweise kovalent gebunden, z.B. an den Linker oder an einen anderen Substitutenten der Liganden L₁, L₂ oder L₃. Der Begriff "Ladungsträger" bedeutet im Sinne der vorliegenden Erfindung eine Gruppe, die bei einem pH-Wert in einem Bereich von 6 bis 8 überwiegend in ionischer Form vorliegt. Der Komplex enthält vorzugsweise bis zu 10, besonders bevorzugt 2 bis 8 solcher Ladungsträger.

Besonders bevorzugt enthält der Komplex mindestens einen negativen Ladungsträger. Beispiele für geeignete negative Ladungsträger sind Phosphat-, Phosphonat-, Sulfonat- und Carboxylatgruppen, wobei Sulfonat- und Carboxylatgruppen am meisten bevorzugt sind.

Beispiele für positive Ladungsträger sind Amino- und ein oder mehrfach substitutierte Aminogruppen wie etwa Mono-, Di- oder Trialkylaminogruppen wobei Alkyl einen geraden oder verzweigten Alkylrest von 1-6 C-Atomen oder einen cyclischen Alkylrest von 3-6 C-Atomen bedeutet.

Der Linker zwischen dem Liganden und der biologischen Substanz hat vorzugsweise eine Kettenlänge von 4 bis 40 Atomen und kann eine durch Einbau von Heteroatomen, z.B. Amidfunktionen, modifizierte Alkylenkette sein.

Beispielsweise kann ein Linker, der freie Ladungsträger enthält, zumindest teilweise aus Aminocarbonsäure-Einheiten aufgebaut sein, die über Peptidbindungen miteinander verknüpft sind. Dabei können die Ladungsträger aus freien Amino- oder/und Carboxylatgruppen von polyfunktionellen Aminocarbonsäuren stammen, die mindestens drei geladene Gruppen (Amino- plus Carboxylat) enthalten, so daß nach Einbau in den Linker und damit verbundener Reaktion von zwei der geladenen Gruppen noch mindestens ein freier Ladungsträger vorhanden ist.

Weiterhin können die freien Ladungsträger aus Substituenten der Liganden stammen, die nicht Bestandteil des Linkers sind. Diese Ladungsträger können direkt oder über eine Spacergruppe an die heterocyclischen Ringe gebunden sein. Der Spacer weist - sofern vorhanden - eine Kettenlänge von vorzugsweise 1-8 Atomen auf und kann eine durch Einbau von Heteroatomen, z.B. Amidfunktionen, modifizierte Alkylenkette sein.

Weiterhin sind für das erfindungsgemäße Verfahren Komplexe geeignet, die eine hydrophile Gruppe enthalten. Beispiele für geeingete hydrophile Gruppen sind C₂-C₃-Alkylenoxy-Einheiten, C₂-C₃-Alkylenthio-Einheiten und Polyhydroxyeinheiten.

Die Polyhydroxy-Einheiten werden vorzugsweise aus Gruppen der Formeln (IIa) oder (IIb) ausgewählt:

-NR-W (IIa)

-O-W- (IIb)

worin
W einen organischen Rest mit mindestens 2 Hydroxygruppen und
R Wasserstoff oder C₁-C₅ Alkyl, vorzugsweise Wasserstoff oder C₁ bis C₃ Alkyl bedeutet.

Der organische Rest W enthält vorzugsweise 2 bis 6 und besonders bevorzugt 2 bis 4 Hydroxygruppen. Weiterhin sollte W günstigerweise 2 bis 10 und insbesondere 3 bis 6 Kohlenstoffatome enthalten. Spezifische Beispiele für geeignete Polyhydroxyeinheiten sind Reste von Polyalkoholen wie etwa Glycerin oder Aminopolyalkoholen. Ein bevorzugter Aminoalkohol ist Tris(2-amino-2-hydroxymethyl)-1,3-propantriol). Die Polyalkohole bzw. Aminopolyalkohole sind an den Metallkomplex vorzugsweise in Form von Estern bzw. Amiden gekoppelt.

Die C₂-C₃-Alkylenoxy bzw. C₂-C₃-Alkylenthio-Einheiten des erfindungsgemäßen Metallkomplexes sind vorzugsweise C₂-Einheiten und insbesondere Ethylenoxy-Einheiten. Der Komplex enthält pro Metallkation vorzugsweise 1 bis 30 und bevorzugt 2 bis 20 Alkylenoxy- bzw. Alkylenthio-Einheiten. Die Alkylenoxy- bzw. Alkylenthio-Einheiten können gegebenenfalls über einen Brückenkopf miteinander verbrückt sein. Andererseits können über einen solchen Brückenkopf auch mehrere Komplexeinheiten miteinander verknüpft sein, wobei Halbkäfig- oder Käfigstrukturen entstehen können.

Konkrete Beispiele für geeignete hydrophile oder/und geladene Metallkomplexe sind in EP-A-0 178 540, WO 96/03409 und WO96/03410 angegeben. Die Herstellung solcher Metallkomplexe kann nach bekannten Methoden erfolgen, beispielsweise durch Reaktion eines Metallsalzes, z.B. eines Metallhalogenids, und gegebenenfalls anschließendem Austausch des Halogenidions durch Hexafluorophosphat-, Trifluoracetat- oder Tetrafluoroborat-Gruppen. Derartige Verfahren sind bekannt. Der Metallkomplex wird für das erfindungsgemäße Verfahren üblicherweise in Form von Konjugaten mit einer biologischen Substanz eingesetzt, wobei an die biologische Substanz mindestens ein Metallkomplex gekoppelt ist. Beispiele für geeignete biologische Substanzen sind Zellen, Viren, subzelluläre Teilchen, Proteine, Lipoproteine, Glycoproteine, Peptide, Polypeptide, Nukleinsäuren, Oligosaccharide, Polysaccharide, Lipopolysaccharide, zelluläre Metaboliten, Haptene, Hormone, pharmakologische Wirkstoffe, Alkaloide, Steroide, Vitamine, Aminosäuren und Zucker.

Die Kopplung des Metallkomplexes mit der biologischen Substanz erfolgt vorzugsweise über eine reaktive oder aktivierbare funktionelle Gruppe am Metallkomplex, z.B. ein Carbonsäurehalogenid, ein Carbonsäureanhydrid oder einen Aktivester wie etwa ein N-Hydroxy-Succinimidester, oder ein Maleimid, die mit einer funktionellen Gruppe der biologischen Substanz kovalent koppeln kann. Wenn die funktionelle Gruppe ein Carbonsäureanhydrid, Carbonsäurehalogenid oder Aktivester ist, kann beispielsweise eine Kopplung mit freien Aminogruppen der biologischen Substanz erfolgen. Wenn die funktionelle Gruppe ein Maleimidrest ist, kann eine Kopplung mit freien SH-Gruppen der biologischen Substanz erfolgen. Auf analoge Weise kann auch eine Aktivierung von funktionellen Gruppen der biologischen Substanz erfolgen, die anschließend beispielsweise mit einer freien Carbonsäure-, Amino- oder Thiolfunktion des Metallkomplexes reagieren können.

Bisherige experimentelle Befunde zeigen, daß die Metallkomplexe das verbesserte Signalverhalten, d.h. ein im wesentlichen konstantes Signal über die gesamte Meßdauer, sowohl als freier Komplex, als auch gekoppelt an eine biologische Substanz, z.B. einen Antikörper (für einen homogenen Assay) oder immobilisiert auf einer Festphase, z.B. paramagnetische Mikrobeads über eine hochaffine Bindung, z.B. eine immunologische bzw. eine Biotin-Strepatvidin-Bindung, zeigen. Unerwartete weitere vorteilhafte Eigenschaften des erfindungsgemäßen Verfahrens sind, daß die unspezifische Bindung eines Konjugats aus einer biologischen Substanz und einem Metallkomplex an die Festphase z.B. Mikrobeads wesentlich geringer als bei bisher verwendeten Metallkomplexen und Verfahrensbedingungen ist. Auch bei der unspezifischen Bindung an die Elektrodenoberfläche werden Verbesserungen gefunden. Darüber hinaus kann das Konjugat aus einem erfindungsgemäßen Metallkomplex und einer biologischen Substanz, beispielsweise in freier Form, bei einer homogenen Nachweisvariante eine höhere Signalamplitude als ein bisher verwendetes Konjugat bei gleicher Konzentration zeigen.

Noch ein weiterer Gegenstand der vorliegenden Erfindung ist ein Verfahren zum Nachweis eines Analyten in einer Probe durch Elektrochemilumineszenz, wobei man als Elektrochemilumineszenz-Markierungsgruppe einen Metallkomplex, der mindestens einen Ladungsträger oder/und mindestens eine hydrophile Gruppe enthält, verwendet und die Messung für eine Dauer von mindestens 0,5 s, vorzugsweise von mindestens 1 s und besonders bevorzugt von mindestens 2 s durchführt. Bevorzugte Merkmale dieses Verfahrens sind oben ausführlich erläutert.

Weiterhin wird die vorliegende Erfindung durch die nachfolgenden Beispiele und Figuren näher verdeutlicht. Es zeigen:
- Fig. 1: einen Vergleich von Lumineszenzmessungen mit freien Rubipy- und Rubaphe-Komplexen,
- Fig. 2: einen Vergleich von Lumineszenzmessungen mit Rubipy- und Rubaphe-Antikörper-Komplexen, und
- Fig. 3: einen möglichen Potentialverlauf in einem Analysezyklus.

### Beispiele

### Beispiel 1 Homogene Testführung

### Materialien

Der Ruthenium(bipyridyl)₃-Komplex (Rubpy) und der Ruthenium(bathophenantrolin(SO₃)₂)₃-Komplex (Rubaphe) wurden hergestellt gemäß EP-A-0 178 450 oder WO 96/03410.

Diese Komplexe wurden an polyklonales Anti-T4-IgG-Antiserum aus Schafen nach Standardmethoden gekoppelt.

Gerät: Elecsys 2010 Seriengerät

Die Testdurchführung erfolgte in einem Analysenzyklus, welcher der Potentialführung des in Beispiel 2 beschriebenen heterogenen Tests im wesentlichen entspricht, abgesehen davon, daß auf die Verwendung von Mikropartikeln und auf eine Bound-Free-Trennung verzichtet wurde. Anstelle des Abfangens (Capturing) der Mikropartikel auf der Elektrodenoberfläche mußte daher ein durch Luftblasen eingegrenztes Lösungssegment mit Markermolekülen über der Elektrode in der Meßkammer positioniert werden. Dann erfolgte die Lumineszenzmessung.

In einem ersten Test wurden die Metallkomplexe Rubpy und Rubaphe jeweils alleine (in Konzentrationen von 10 nM) bzw. als Antikörper-Konjugate 1:15 (133 ng) in einer homogenen Testführung untersucht. Die Dauer des Auswerteintervalls betrug 400 ms bzw. 1,2 s.

Die Ergebnisse dieser Tests sind in Tabelle 1 zusammengefaßt.

**Tabelle 1**

| homogener Test auf Elecsys 2010 Seriengerät | Signalausbeute bei 400 ms langem Auswerteintervall (0,8-1,2 s) | Signalausbeute bei 1,2 s langem Auswerteintervall (0,8-2,0 s) | VerbesserungsFaktor Standardzu längerem Auswerteintervall |
|---|---|---|---|
| Rubpy (Label allein) | 115185 counts | 270027 conts | 2,3 |
| Rubaphe (Label allein) | 117212 counts | 338435 counts | 2,8 |
| Verbesserungs-Faktor Rubaphe/Ruby | 1,02 | 1,3 | |
| Rubpy (Antikörper-Konjugat 1:15) | 29119 counts | 72326 counts | 2,4 |
| Rubaphe (Antikörper-Konjugat) | 46647 counts | 136719 counts | 2,9 |
| Verbesserungs-Faktor Rubaphe/Rubpy | 1,6 | 1,9 | |

Im Falle der homogenen Testführung wird bei der Verwendung von Rubaphe eine Signalsteigerung gegenüber Rubpy gefunden. Insbesondere bei einem längeren Auswerteintervall ergibt Rubaphe ein signifikant besseres Signal als Rubpy.

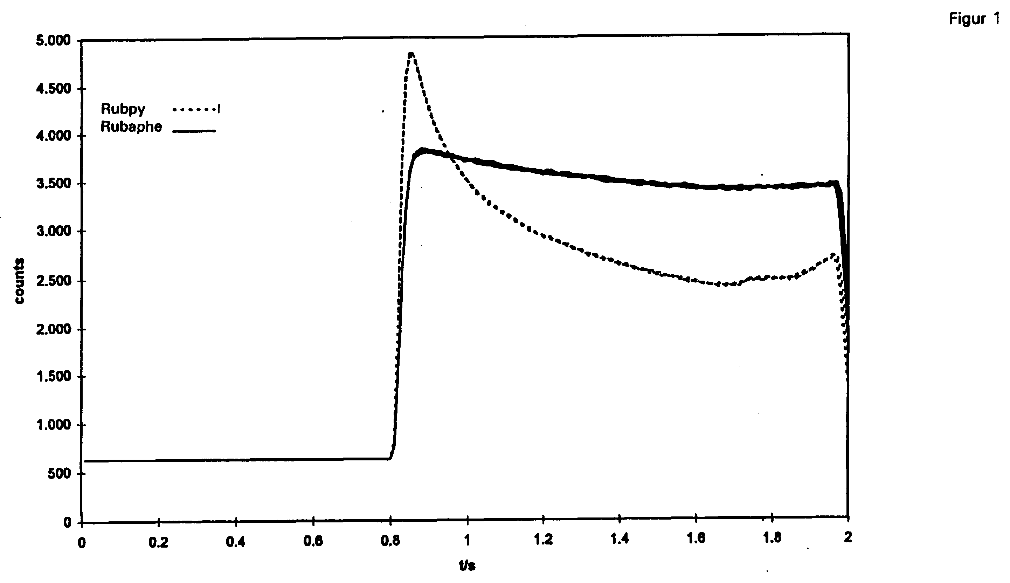

In Fig. 1 ist das graphische Ergebnis einer Messung mit freiem Komplex in Analysepuffer dargestellt. Es ist ersichtlich, daß Rubaphe einen über die Gesamtdauer des Signals im wesentlichen konstanten Signalverlauf aufweist, während bei Rubpy bereits nach wenigen Millisekunden eine deutliche Signalabnahme erfolgt.

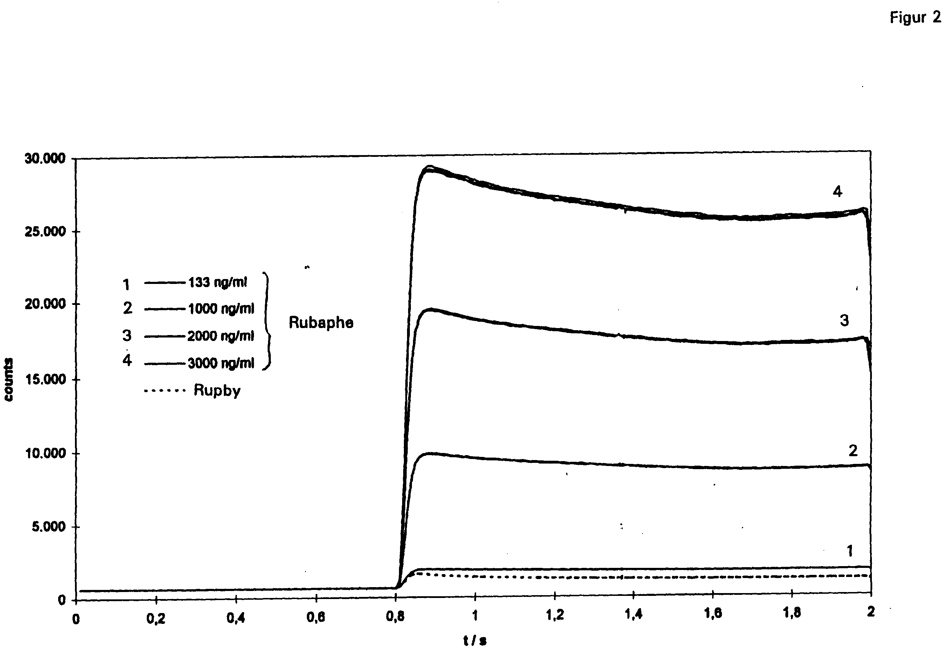

Fig. 2 zeigt das Ergebnis einer Messung mit Antikörper-Metallkomplexkonjugaten. Auch dort ist die Überlegenheit von Rubaphe gegenüber Rubipy zu erkennen.

### Beispiel 2 Heterogene Testführung mit Mikropartikeln

Es wurde ein heterogener kompetitiver Test auf Progesteron 2 G durchgeführt. Hierzu wurden 85 µl eines biotinylierten monoklonalen Anti-Progesteron-IgG-Antikörpers (60 ng/ml) mit jeweils 75 µl Rubipy- bzw. Rubaphe-Progesteron-Konjugat (20 ng/ml) und 10 µl Streptavidin-beschichteten Mikropartikeln versetzt. Die Herstellung der Progesteron-Rutheniumkomplex-Konjugate erfolgte analog WO 96/03410 durch Kopplung eines aktivierten Metallkomplexes (N-Hydroxysuccinimidester) an Progesteron-3 CMO-aminodioxaoctan in DMF. Die Gesamtinkubationsdauer für diesen Schritt betrug 8,4 min. Dann wurden 30 µl der zu testenden Probe zugesetzt und weiter für 8,4 min inkubiert; dann erfolgt die Lumineszenzmessung.

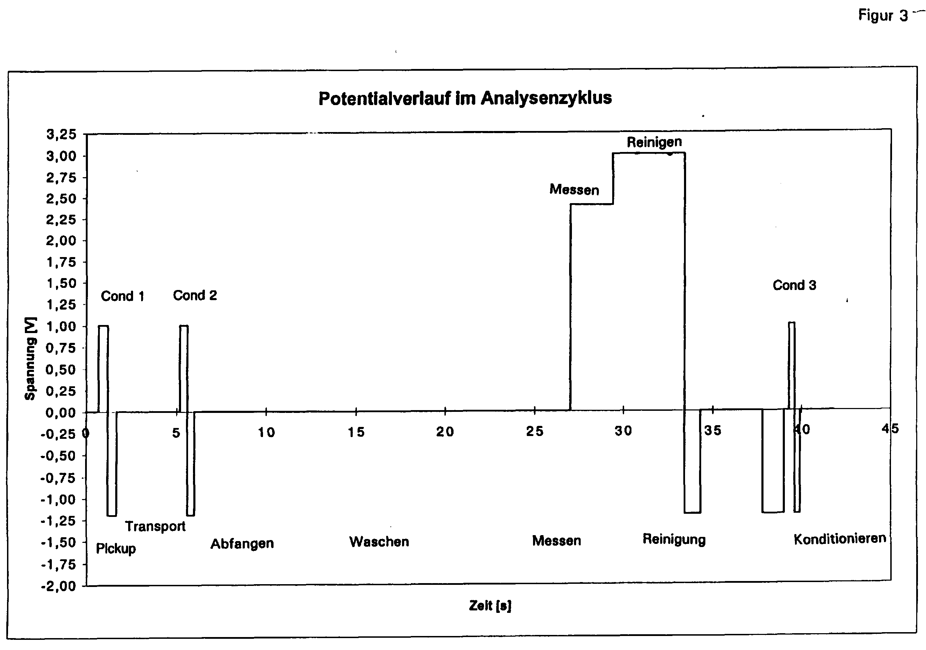

Der Ablauf eines Analysenzyklus anhand des Potentialprofils ist in Fig. 3 gezeigt. Die als Cond 1, Cond 2 und Cond 3 bezeichneten Konditionierungsschritte, insbesondere Cond 2 mit dem darin enthaltenen reduktiven Teil (negative Spannung bezüglich der Ag/AgCl-Referenz) sind von großer Bedeutung für die Konditionierung der Elektrode und für ein kontinuierliches Signal mit den getesteten Metallkomplexen.

Das Ergebnis für Rubpy und Rubaphe ist in Tabelle 2 angegeben.

**Tabelle 2**

| Calibrator | Konzentration Progesteron [ng/ml] | Rubaphe Signale | Rubpy Signale |
|---|---|---|---|
| Cal 1 | 0 | 160893 | 257102 |
| Cal 2 | 2 | 116140 | 193590 |
| Cal 3 | 5 | 78362 | 142102 |
| Cal 4 | 10 | 52570 | 101752 |
| Cal 5 | 20 | 43306 | 89240 |
| Cal 6 | 70 | 33806 | 67518 |
| Cal 7 | 100 | 31446 | 62772 |

Für Rubpy ergibt sich in Abwesenheit von Progesteron zwar ein um 60% höheres Maximalsignal. Da das minimale Signal (100 ng/ml Progesteron) bei Rubpy um ca. 200% höher als Rubaphe ist, ergibt sich für Rubaphe eine um mindestens 20% bessere Signadynamik (Verhältnis von maximaler Signalstärke zu minimaler Signalstärke).

## Patentansprüche

1. Verfahren zum Nachweis eines Analyten in einer Probe durch Elektrochemilumineszenzmessung, umfassend die Schritte:
(a) Bereitstellen einer Elektrochemilumineszenzvorrichtung umfassend eine Meßelektrode,
(b) Inkontaktbringen einer Konditionierungsflüssigkeit, die ein Elektrochemilumineszenz-Cosubstrat enthält, mit der Elektrode,
(c) Einstellen von Bedingungen an der Elektrode, welche zur Ausbildung einer aktivierte Moleküle des Elektrochemilumineszenz-Cosubstrats enthaltenden Schicht auf oder/und im Grenzschichtbereich der Elektrode führen,
(d) Inkontaktbringen der Probe, die einen Metallkomplex, der mindestens einen Ladungsträger oder/und mindestens eine hydrophile Gruppe enthält, als Elektrochemilumineszenz-Markierungsgruppe sowie ein Elekrochemilumineszenz-Cosubstrat enthält, mit der Elektrode,
(e) Anlegen eines Potentials an die Elektrode, das den Ablauf einer Elektrochemilumineszenzreaktion ermöglicht, und Messen der Elektrochemilumineszenz und
(d) Korrelieren der gemessenen Luminesenz mit dem Vorhandensein bzw. der Menge des Analyten in der Probe.

2. Verfahren nach Anspruch 1,
**dadurch gekennzeichnet,**
daß man einen Metallkomplex als Markierungsgruppe verwendet, der eine Struktur der allgemeinen Formel (I) enthält:
[M(L₁L₂L₃)]ₙ-Yₘ- (I)
worin
- M ein zwei- oder dreiwertiges Metallkation ausgewählt aus Seltenerde- oder Übergangsmetallkationen ist,
- L₁, L₂ und L₃ gleich oder verschieden sind und Liganden mit mindestens zwei stickstoffhaltigen Heterocyclen bedeuten, wobei L₁, L₂ und L₃ über Stickstoffatome an das Metallkation gebunden sind,
- Y einen an einen der Liganden gebundenen Linker bedeutet,
- m eine ganze Zahl von 1 bis 10 ist,
- n eine ganze Zahl von 1 bis 6 ist und
- mindestens eine hydrophile Gruppe oder/und ein Ladungsträger im Komplex vorhanden ist.

3. Verfahren nach Anspruch 2,
**dadurch gekennzeichnet,**
daß die hydrophile Gruppe oder/und der Ladungsträger an einen Linker oder an einen anderen Substituenten der Liganden L₁, L₂ oder L₃ gebunden sind.

4. Verfahren nach einem der Ansprüche 1 bis 3,
**dadurch gekennzeichnet,**
daß man einen Komplex verwendet, der mindestens einen negativen Ladungsträger ausgewählt aus Phosphat-, Phosphonat-, Sulfonat- und Carboxylatgruppen enthält.

5. Verfahren nach einem der Ansprüche 1 bis 4,
**dadurch gekennzeichnet,**
daß man einen Komplex verwendet, der mindestens eine hydrophile Gruppe ausgewählt aus C₂-C₃-Alkylenoxy-Einheiten, C₂-C₃-Alkylenthio-Einheiten und Polyhydroxy-Einheiten enthält.

6. Verfahren nach einem der Ansprüche 1 bis 5,
**dadurch gekennzeichnet,**
daß man als Elektrochemilumineszenz-Cosubstrat ein Trialkylamin verwendet, wobei die Alkylreste jeweils unabhängig 1-4 C-Atome enthalten.

7. Verfahren nach einem der Ansprüche 1 bis 6,
**dadurch gekennzeichnet,**
daß in Schritt (c) ein Potential von -0,3 bis -1,2 V (bezogen auf eine Ag/AgCl-Referenzelektrode) angelegt wird.

8. Verfahren nach einem der Ansprüche 1 bis 7,
**dadurch gekennzeichnet,**
daß in Schritt (e) während der gesamten Messdauer ein Potential angelegt wird, welches ausreicht, die Lumineszenzreaktion aufrecht zu erhalten.

9. Verfahren nach einem der Ansprüche 1 bis 7,
**dadurch gekennzeichnet,**
daß in Schritt (e) ein posititves Elektrodenpotential von mindestens + 1,2 V (bezogen auf eine Ag/AgCl-Referenzelektrode) angelegt wird.

10. Verfahren zum Nachweis eines Analyten in einer Probe durch Elektrochemilumineszenz,
**dadurch gekennzeichnet,**
daß man als Elektrochemilumineszenz-Markierungsgruppe einen Metallkomplex, der mindestents einen Ladungsträger oder/und mindestens eine hydrophile Gruppe enthält, verwendet und die Messung für die Dauer von mindestens 0,5 s durchführt.
